(19) Europäisches Patentamt
European Patent Office
Office européen des brevets

(11) Veröffentlichungsnummer: **0 144 885**
**B1**

(12) # EUROPÄISCHE PATENTSCHRIFT

(45) Veröffentlichungstag der Patentschrift: **26.07.89**

(51) Int. Cl.⁴: **C 07 C 120/00**, C 07 C 103/133

(21) Anmeldenummer: **84114259.9**

(22) Anmeldetag: **26.11.84**

(54) **Verfahren zur gleichzeitigen Herstellung von Nitrilen und Acrylamid bzw. Methacrylamid.**

(30) Priorität: **02.12.83 DE 3343673**

(43) Veröffentlichungstag der Anmeldung: **19.06.85 Patentblatt 85/25**

(45) Bekanntmachung des Hinweises auf die Patenterteilung: **26.07.89 Patentblatt 89/30**

(84) Benannte Vertragsstaaten: **BE CH DE FR GB IT LI NL**

(56) Entgegenhaltungen:
**EP-A-0 144 883**
**FR-A-2 118 517**

**SYNTHESIS, Nr. 9, September 1983, Seiten 741,742; O. ATTANASI et al.: "Effect of metal ions in organic synthesis; XVII. Mild, easy, and high-yield conversion of aldoximes into nitriles under copper(II) acetate-catalysis"**
**CHEMICAL ABSTRACTS EIGHTH COLLECTIVE INDEX, Bände 66-75, 1967-1971, American Chemical Society, Columbus, Ohio, US, Seite 259S, Acetic acid, compounds, Spalte 3, copper(2) salt [142-71-2]; Seite 260S, Spalte 1, catalysts, for acrylamide manuf., 73: P 77888k; & CHEMICAL ABSTRACTS, Band 73, Nr. 16, 19. Oktober 1970, Zusammenfassung Nr. 77888k, Columbus, Ohio, US; & JP-A-70 21 295 (ASAHI CHEMICAL INDUSTRY CO. LTD.) 18-07-1970**

(73) Patentinhaber: **BASF Aktiengesellschaft, Carl-Bosch-Strasse 38, D-6700 Ludwigshafen (DE)**

(72) Erfinder: **Merger, Franz, Dr.-Chem., Max-Slevogt-Strasse 25, D-6710 Frankenthal (DE)**
Erfinder: **Schwarz, Wolfgang, Dr.-Chem., Wesostrasse 132, D-7507 Pfinztal (DE)**

**EP 0 144 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur gleichzeitigen Herstellung von Nitrilen und Acryl- bzw. Methacrylamid, ausgehend von den Oximen der jeweiligen Aldehyde und Acryl- bzw. Methacrylnitril.

In der Literatur sind zahlreiche Verfahren zur Herstellung von Nitrilen beschrieben worden, so sollen beispielsweise nach dem Vorschlag der DE-A-2 310 184 Carbonsäureamide mittels Phosgen in die entsprechenden Nitrile übergeführt werden. Dieses Verfahren ist aber, bedingt durch den Umgang mit Phosgen, besonders aufwendig.

Spezielle Nitrile, wie Acrylnitril oder aromatische Nitrile, können durch Ammonoxidation von Propen bzw. Propan oder der entsprechenden Alkylaromaten hergestellt werden (Hydrocarbon Process. 41 (11), 187 (1962), DE-B-2 057 986 oder DE-A-1 643 722). Allerdings ist bereits die Herstellung von Methacrylnitril nach diesem Prinzip wenig befriedigend, und die Ammonoxidation höherer Olefine ist nicht bekannt.

Auch die Dehydratisierung der gut zugänglichen Aldoxime zu Nitrilen ist beschrieben (Houben-Weyl «Methoden der organischen Chemie» 8, 235f.). Die am häufigsten verwendete wasserabspaltende Verbindung ist dabei Acetanhydrid, das jedoch bei säureempfindlichen Verbindungen Nebenreaktionen verursacht und teilweise auch acylierend wirken kann.

Thionylchlorid kommt aus technischen Gründen wegen der Entstehung von Schwefeldioxid und Chlorwasserstoff für diesen Zweck nicht in Betracht, darüber hinaus neigt es ebenfalls zu Nebenreaktionen.

Andere Dehydratisierungsmittel, z.B. Cyanurchlorid (US-A-3 928 274) werden nur in wenigen speziellen Fällen verwendet.

Ferner ist aus Synthesis 9 (1983), 741–742 bekannt, daß man Nitrile aus Aldoximinen in Gegenwart von Kupfer-(II)-acetatmonohydrat erhalten kann.

Aufgabe der vorliegenden Erfindung war es, ein Verfahren bereitzustellen, das geeignet ist, in einfacher Weise und ohne großen technischen Aufwand, eine Vielzahl von Nitrilen zu synthetisieren.

Da das bei der Dehydratisierung der Aldoxime in die Nitrile anfallende Wasser mit den jeweiligen Dehydratisierungsmitteln im allgemeinen zu größeren Mengen nicht verwertbarer Abfallprodukte führt, war es eine weitere Aufgabe der Erfindung, durch die Verwendung geeigneter Wasser aufnehmender Reaktionspartner neben den Nitrilen zusätzlich weitere Wertprodukte herzustellen.

Es wurde nun gefunden, daß man gleichzeitig Nitrile der allgemeinen Formel I

$$R^1\text{–}CN \qquad (I),$$

in der $R^1$ einen gesättigten oder ungesättigten, geradkettigen, verzweigten oder cyclischen Alkylrest, einen Aralkylrest oder einen Arylrest mit jeweils bis zu 20 Kohlenstoffatomen bedeutet, der gegebenenfalls weiter substituiert sein kann, und Acrylamide der allgemeinen Formel II

$CH_2=C\text{–}CONH_2$ (II),
|
$R^2$

in der $R^2$ ein Wasserstoffatom oder den Methylrest bedeutet, vorteilhaft erhält, wenn man Aldoxime der allgemeinen Formel III

$$R^1\text{–}CH=NOH \qquad (III),$$

in der $R^1$ die oben genannte Bedeutung besitzt, in Gegenwart eines Kupfer(II)carboxylats einer Carbonsäure mit 2 bis 18 Kohlenstoffatomen sowie in Anwesenheit von 150 bis 500 Mol%, bezogen auf das Oxim, eines Acrylnitrils der allgemeinen Formel IV

$CH_2=C\text{–}CN$ (IV),
|
$R^2$

in der $R^2$ die oben genannte Bedeutung besitzt, auf eine Temperatur von 50 bis 180 °C erwärmt.

Das erfindungsgemäße Verfahren wird mit Oximen durchgeführt, die sich von Aldehyden der allgemeinen Formel VI ableiten

$$R^1\text{–}CHO \qquad (VI),$$

in der $R^1$ einen gesättigten oder ungesättigten, geradkettigen, verzweigten oder cyclischen Alkylrest, einen Aralkylrest oder einen Arylrest mit jeweils bis zu 20 Kohlenstoffatomen bedeutet. Dieser Rest kann durch weitere Gruppen, die sich unter den Reaktionsbedingungen indifferent verhalten, substituiert sein, z.B. durch Halogen, niedere Alkoxygruppen (auch in geminaler Stellung), niedere Acyloxygruppen, niedere Alkoxycarbonylgruppen oder niedere Mono- und Dialkylaminogruppen.

Solche Aldehyde sind beispielsweise Acetaldehyd, Propionaldehyd, Acrolein, Butyraldehyd, 3-Methylbut-2-enal, 2-Methylpentanal, 2-Ethylhexanal, Citronellal, Citral, 2-Phenylpropanal, Benzaldehyd, 4-Methylbenzaldehyd, 4-Chlorbenzaldehyd, 4-Methoxybenzaldehyd oder 4-Dimethylaminobenzaldehyd.

Vorzugsweise führt man das erfindungsgemäße Verfahren mit Aldoximen der allgemeinen Formel V

$CH_2=C\text{–}CH=NOH$ (V),
|
$R^3$

in der $R^3$ einen geradkettigen Alkylrest mit 2 bis 15 Kohlenstoffatomen oder einen verzweigten oder cyclischen Alkylrest mit bis zu 15 Kohlenstoffatomen bedeutet, der gegebenenfalls weiter substituiert sein kann, durch. Als weitere Substituenten kommen beispielsweise die oben genannten Gruppen in Betracht.

Die Aldoxime der allgemeinen Formel V leiten sich von den jeweiligen alpha-alkylsubstituierten Acroleinen ab. Solche Acroleine sind z.B. alpha-Ethylacrolein, alpha-Butylacrolein, alpha-(2-Ethylhexyl)acrolein, alpha-Nonylacrolein, alpha-Cyclohexylacrolein, alpha-(4-Methylcyclohexyl)-acrolein, alpha-(3-Carbethoxypropyl)acrolein oder alpha-(4,4-Dimethylaminobutyl)acrolein.

Die Herstellung der genannten Aldehyde geschieht nach an sich bekannten Methoden und ist für die Erfindung ohne Belang.

Die für das erfindungsgemäße Verfahren geeigneten Aldoxime lassen sich ebenfalls nach an sich bekannten Methoden, wie sie beispielsweise in Houben-Weyl «Methoden der organischen Chemie», Band 10/4, S. 55f. beschrieben sind, aus den jeweiligen Aldehyden und Hydroxylammoniumsalzen herstellen.

Das erfindungsgemäße Verfahren wird bei einer Temperatur von 50 bis 180 °C, vorzugsweise 75 bis 150 °C und insbesondere 100 bis 140 °C, durchgeführt.

Im allgemeinen erfolgt die Umsetzung bei atmosphärischem Druck. In manchen Fällen ist es auch vorteilhaft, sie unter erhöhtem Druck (bis zu 20 bar) vorzunehmen.

Man kann dabei lösungsmittelfrei oder aber vorzugsweise in Gegenwart eines inerten Lösungsmittels, wie Toluol, Xylol, Mesitylen, Chlorbenzol, Nitrobenzol, Tetralin, Decalin, Dioxan, Dibutylether oder Essigsäure-n-butylester, arbeiten.

Die erfindungsgemäße gleichzeitige Herstellung von Nitrilen und von Acrylamid oder Methacrylamid wird in Gegenwart von Kupfer(II)carboxylaten vorgenommen, die sich von Carbonsäuren mit 2 bis 18 Kohlenstoffatomen, vorzugsweise 4 bis 12 Kohlenstoffatomen und insbesondere 6 bis 10 Kohlenstoffatomen, ableiten.

Als solche Säuren kommen (gegebenenfalls substituierte) Carbonsäuren mit einem gesättigten oder ungesättigten geradkettigen, verzweigten oder cyclischen Alkylrest, Aralkansäuren, Oxaalkansäuren sowie aromatische Carbonsäuren in Betracht, beispielsweise Essigsäure, Propionsäure, alpha-Chlorpropionsäure, Isobuttersäure, 2-Methylbutansäure, 2-Ethylhexansäure, 3,5,5-Trimethylhexansäure, Decansäure, 9-Decensäure, 9-Dodecensäure, 9-Octadecensäure, Cyclohexancarbonsäure, Phenylessigsäure, 1-Phenylcyclopentan-1-carbonsäure, Methoxyessigsäure, Benzoesäure, 3,5-Dichlorbenzoesäure oder Naphthoesäure.

Die Herstellung der für das erfindungsgemäße Verfahren benötigten Kupfersalze kann nach an sich bekannten Methoden, z.B. durch Umsetzung der Carbonsäuren mit Kupfer(II)carbonat oder durch Umsetzung von Kupfersalzen mit den Alkali- oder Ammoniumsalzen der jeweiligen Carbonsäure, erfolgen.

Die Menge des eingesetzten Kupfer(II)carboxylats beträgt 0,1 bis 10 Mol%, bevorzugt 0,1 bis 5 Mol%, und insbesondere 0,5 bis 1,5 Mol%, jeweils bezogen auf 1 Mol Aldoxim.

Im erfindungsgemäßen Verfahren ist außerdem die Anwesenheit von Acrylnitril oder Methacrylnitril (siehe oben, allgemeine Formel IV) erforderlich.

Man setzt dabei Acrylnitril bzw. Methacrylnitril bevorzugt in Mengen von 150 bis 300 Mol%, jeweils bezogen auf 1 Mol Aldoxim, zu.

Unter diesen Bedingungen entsteht aus dem eingesetzten Aldoxim in guten Ausbeuten (bis zu 95% der Theorie) das gewünschte Nitril, gleichzeitig wird die dem Oxim entsprechende Menge des zugesetzten Acrylnitrils bzw. Methacrylnitrils nahezu quantitativ in das betreffende Acrylamid übergeführt.

Das erfindungsgemäße Verfahren wird üblicherweise so ausgeführt, daß man Kupfer(II)carboxylat und Acrylnitril oder Methacrylnitril, vorzugsweise in einem inerten Lösungsmittel, auf den genannten Temperaturbereich erwärmt. Anschließend gibt man das jeweilige Aldoxim zu. Dabei ist sowohl eine diskontinuierliche als auch eine kontinuierliche Arbeitsweise möglich.

Nach Beendigung der Reaktion wird zunächst das Zielprodukt Acrylamid bzw. Methacrylamid (siehe oben, allgemeine Formel II) durch Kristallisation abgetrennt. (Man erhält dabei üblicherweise ca. 95 bis 100 Mol% der dem Oxim entsprechenden Menge des zugesetzten Acrylnitrils bzw. Methacrylnitrils in Form des Amids zurück.) Die resultierende Lösung des anderen Zielproduktes (Nitril) wird dann vom Lösungsmittel und überschüssigem Acrylnitril bzw. Methacrylnitril befreit.

Die so erhaltenen Nitrile besitzen nach dem Auswaschen des Katalysators mit Wasser für viele Verwendungszwecke einen ausreichenden Reinheitsgrad. Sie können aber auch durch fraktionierte Destillation weiter gereinigt werden oder gegebenenfalls durch Ionenaustausch von Kupferspuren befreit werden.

Auch das gewünschte Acrylamid bzw. Methacrylamid kann für viele Zwecke direkt verwendet werden. Es kann aber ebenfalls weiter gereinigt werden (Umkristallisation, Ionenaustausch).

Mittels des erfindungsgemäßen Verfahren ist es möglich, auf einfachem Wege auch solche Nitrile herzustellen, deren Synthese üblicherweise mit Schwierigkeiten verbunden ist.

Die nach dem erfindungsgemäßen Verfahren gewonnenen Nitrile sind wertvolle Zwischenprodukte, beispielsweise für die Synthese von Pharmazeutika und Farbstoffen oder für die Herstellung von modifizierbaren Polymeren, z.B. für Dispersionen und Lacke.

Acrylamid dient u.a. als Ausgangsprodukt für Mischpolymerisate. Methacrylamid findet z.B. als Comonomeres, hauptsächlich bei der Dispersions-Herstellung Verwendung.

Die Erfindung soll durch folgende Beispiele veranschaulicht werden.

Beispiel 1

Eine Lösung von 132,5 g Acrylnitril und 2 g Kupfer(II)acetat in 320 g o-Xylol wurde zum Sieden erhitzt (92 °C) und anschließend wurden innerhalb von 15 min 85 g Methacroleinoxim zugetropft, wobei die Temperatur auf 108 °C anstieg. Nach dem Abkühlen auf Raumtemperatur wurde der Reaktionsaustrag gaschromatographisch analysiert

(Methode des internen Standards). Danach enthielt die Lösung 63 g (94%) Methacrylnitril, 84,4 g Acrylnitril und 61,8 g Acrylamid (= 96% bezogen auf umgesetztes Acrylnitril).

Beispiel 2

Eine Lösung von 382 g Acrylnitril und 25,2 g Kupfer(II)-2-ethylhexanoat in 1800 g o-Xylol wurde zum Sieden erhitzt (92 °C) und anschließend wurden innerhalb von 50 min 375 g alpha-Ethylacroleinoxim (Reinheit 95%) zugetropft. Dabei stieg die Temperatur bis auf 110 °C an. Man rührte noch 15 min nach, ließ erkalten und saugte das ausgefallene Acrylamid ab. Fraktionierende Destillation der Mutterlauge lieferte 260 g (89%) alpha-Ethylacrylnitril vom Siedepunkt 112 °C.

Beispiel 3

Eine Lösung von 80,7 g Acrylnitril und 5,3 g Kupfer(II)-2-ethylhexanoat in 250 g o-Xylol wurde zum Sieden erhitzt (97 °C) und anschließend wurden innerhalb von 15 min 151,5 g alpha-Nonylacroleinoxim (Reinheit 99%) zugetropft. Die Temperatur stieg während der Zugabe auf 115 °C. Nach beendeter Zugabe wurde noch 10 min unter Rückfluß gekocht, dann kühlte man das Reaktionsgemisch auf − 8 °C ab und saugte das ausgefallene Acrylamid ab. Fraktionierende Destillation der Mutterlauge ergab 121 g (89%) alpha-Nonylacrylnitril vom Siedepunkt 122–124 °C/12 mbar.

Beispiel 4

Eine Lösung von 123 g Hydroxypivalaldoxim (Reinheit 95%) in 100 g o-Xylol wurde innerhalb von 8 min zu einer siedenden Lösung von 106 g Acrylnitril und 7 g Kupfer(II)-2-ethylhexanoat in 410 g o-Xylol getropft, wobei die Temperatur von 96 auf 115 °C anstieg. Man rührte noch 10 min bei 115 °C nach, kühlte auf − 14 °C ab und trennte das ausgefallene Acrylamid ab. Fraktionierende Destillation lieferte 82 g (83%) Hydroxypivalonitril vom Siedepunkt 95–97 °C/15 mbar.

Beispiel 5

Ein Gemisch von 130 g Acrylnitril und 2,3 g Kupfer(II)-2-ethylhexanoat wurde zum Sieden erhitzt (75 °C) und anschließend wurden innerhalb von 10 min 43 g Methoxypivalinaldoxim zugetropft, wobei die Temperatur auf 83 °C anstieg. Nach der Zugabe rührte man 10 min ohne Erwärmen nach und kühlte dann auf Raumtemperatur ab. Der Acrylnitrilüberschuß wurde abdestilliert, der Destillationsrückstand mit 250 ml Petrolether versetzt und auf − 5 °C abgekühlt. Das ausgefallene Acrylamid wurde abgesaugt und die Mutterlauge im Vakuum eingeengt. Destillation des Rückstands ergab 31,5 g (85%) Methoxypivalonitril vom Siedepunkt 158–160 °C.

Beispiel 6

Eine Lösung von 74,2 g Acrylnitril und 4,9 g Kupfer(II)-2-ethylhexanoat in 250 g o-Xylol wurde zum Sieden erhitzt (92 °C) und anschließend wurden innerhalb von 20 min 84,7 g Benzaldoxim zugetropft. Nach beendeter Zugabe rührte man noch 10 min nach, kühlte dann auf − 5 °C ab und saugte das ausgefallene Acrylamid ab. Fraktionierende Destillation der Mutterlauge ergab 63,5 g (88%) Benzonitril vom Siedepunkt 73 °C/20 mbar.

Beispiel 7

Eine Lösung von 53 g Acrylnitril und 1,0 g Kupfer(II)acetat in 100 g o-Xylol wurde zum Sieden erhitzt (85 °C) und anschließend wurde unter weiterem Erwärmen eine Lösung von 82 g p-Dimethylaminobenzaldoxim in 110 g o-Xylol zugetropft. Der Siedepunkt des Reaktionsgemisches stieg während der Zugabe auf 135 °C. Man rührte noch 30 min unter Rückfluß nach und ließ dann erkalten. Das ausgefallene Acrylamid wurde abgesaugt und die Mutterlauge mit wenig Wasser ausgeschüttelt. Die organische Phase wurde eingeengt und der Rückstand in 400 ml Petrolether aufgenommen. Nach Stehen über Nacht bei 0 °C wurden 66 g (90%) p-Dimethylaminobenzonitril erhalten. Gelbe Kristalle vom Schmelzpunkt 75–76 °C.

Beispiel 8

Eine Lösung von 28 g alpha-(1-Phenylethyl)-acroleinoxim in 10 g o-Xylol wurde innerhalb von 6 min zu einer siedenden Lösung von 17 g Acrylnitril und 0,38 g Kupfer(II)butyrat in 60 g o-Xylol zugetropft. Nach Abklingen der stark exothermen Reaktion wurde auf Raumtemperatur abgekühlt und das auskristallisierte Acrylamid abgesaugt. Die Mutterlauge wurde mit 100 ml Diethylether verdünnt und zweimal mit je 50 ml Wasser extrahiert. Nach dem Trocknen über Magnesiumsulfat engte man die organische Phase am Rotationsverdampfer ein. Der Rückstand wurde fraktionierend destilliert. Man erhielt 22,6 g (90%) alpha-(1-Phenylethyl)acrylnitril vom Siedepunkt 63–65 °C/0,05 mbar.

**Patentansprüche**

1. Verfahren zur gleichzeitigen Herstellung von Nitrilen der allgemeinen Formel I

$$R^1\text{–CN} \qquad (I),$$

in der $R^1$ einen gesättigten oder ungesättigten, geradkettigen, verzweigten oder cyclischen Alkylrest, einen Aralkylrest oder einen Arylrest mit jeweils bis zu 20 Kohlenstoffatomen bedeutet, der gegebenenfalls weiter substituiert sein kann, und von Acrylamiden der allgemeinen Formel II

$$CH_2{=}\underset{\displaystyle R^2}{\underset{|}{C}}\text{–}CONH_2 \qquad (II),$$

in der $R^2$ ein Wasserstoffatom oder den Methylrest bedeutet, dadurch gekennzeichnet, daß man Aldoxime der allgemeinen Formel III

$$R^1\text{–}CH{=}NOH \qquad (III),$$

in der $R^1$ die oben genannte Bedeutung besitzt, in Gegenwart eines Kupfer(II)carboxylats einer Carbonsäure mit 2 bis 18 Kohlenstoffatomen sowie in Anwesenheit von 150 bis 500 Mol%, bezogen auf das Oxim, eines Acrylnitrils der allgemeinen Formel

$$CH_2{=}C(R^2){-}CN \qquad (IV),$$

in der $R^2$ die oben genannte Bedeutung besitzt, auf eine Temperatur von 50 bis 180 °C erwärmt.

2. Verfahren gemäß Anspruch 1, dadurch gekennzeichnet, daß man die Umsetzung bei einer Temperatur von 75 bis 150 °C durchführt.

3. Verfahren gemäß Anspruch 1, dadurch gekennzeichnet, daß man die Umsetzung in Gegenwart eines Kupfer(II)carboxylats einer Carbonsäure mit 4 bis 12 Kohlenstoffatomen durchführt.

4. Verfahren gemäß Anspruch 1, dadurch gekennzeichnet, daß man die Umsetzung in Anwesenheit eines inerten Lösungsmittels durchführt.

5. Verfahren gemäß Anspruch 1, dadurch gekennzeichnet, daß man die Umsetzung mit Aldoximen der allgemeinen Formel V

$$CH_2{=}C(R^3){-}CH{=}NOH \qquad (V),$$

in der $R^3$ einen geradkettigen Alkylrest mit 2 bis 15 Kohlenstoffatomen oder einen verzweigten oder cyclischen Alkylrest mit bis zu 15 Kohlenstoffatomen bedeutet, der gegebenenfalls weiter substituiert sein kann, durchführt.

**Claims**

1. A process for the simultaneous preparation of a nitrile of the formula I

$$R^1{-}CN \qquad (I)$$

where $R^1$ is a saturated or unsaturated, straight-chain, branched or cyclic alkyl radical, an aralkyl radical or an aryl radical, each of which is of not more than 20 carbon atoms and can be unsubstituted or further substituted, and an acrylamide of the formula II

$$CH_2{=}C(R^2){-}CONH_2 \qquad (II)$$

where $R^2$ is hydrogen or methyl, wherein an aldoxime of the formula III

$$R^1{-}CH{=}NOH \qquad (III)$$

where $R^1$ has the above meanings, is heated at from 50 to 180 °C in the presence of a copper(II) salt of a carboxylic acid of 2 to 18 carbon atoms, and in the presence of from 150 to 500 mol%, based on the oxime, of an acrylonitrile of the formula

$$CH_2{=}C(R^2){-}CN \qquad (IV)$$

where $R^2$ has the above meanings.

2. A process as claimed in claim 1, wherein the reaction is carried out at from 75 to 150 °C.

3. A process as claimed in claim 1, wherein the reaction is carried out in the presence of a copper(II) salt of a carboxylic acid of 4 to 12 carbon atoms.

4. A process as claimed in claim 1, wherein the reaction is carried out in the presence of an inert solvent.

5. A process as claimed in claim 1, wherein the reaction is carried out using an aldoxime of the formula V

$$CH_2{=}C(R^3){-}CH{=}NOH \qquad (V)$$

where $R^3$ is a straight-chain alkyl radical of 2 to 15 carbon atoms or a branched or cyclic alkyl radical of not more than 15 carbon atoms which can be unsubstituted or further substituted.

**Revendications**

1. Procédé de préparation simultanée de nitriles de formule générale I

$$R^1{-}CN \qquad (I),$$

dans laquelle $R^1$ est un reste alkyle saturé ou insaturé linéaire, ramifié ou cyclique, aralkyle ou aryle ayant à chaque fois jusqu'à 20 atomes de carbone, qui peut éventuellement être en outre substitué, et d'acrylamides de formule générale II

$$CH_2{=}C(R^2){-}CONH_2 \qquad (II),$$

dans laquelle $R^2$ est un atome d'hydrogène ou un reste méthyle, caractérisé en ce qu'on chauffe à une température de 50 à 180 °C des aldoximes de formule générale III

$$R^1{-}CH{=}NOH \qquad (III),$$

dans laquelle $R^1$ a la signification donnée précédemment, en présence d'un carboxylate de cuivre-(II) d'un acide carboxylique de 2 à 18 atomes de carbone ainsi qu'en présence de 150 à 500% en moles, par rapport à l'oxime, d'un acrylonitrile de formule générale

$$CH_2{=}C(R^2){-}CN \qquad (IV),$$

dans laquelle $R^2$ a la signification donnée précédemment.

2. Procédé selon la revendication 1, caractérisé en ce qu'on effectue la réaction à une température de 75 à 150 °C.

3. Procédé selon la revendication 1, caractérisé en ce qu'on effectue la réaction en présence d'un carboxylate de cuivre-(II) d'un acide carboxylique de 4 à 12 atomes de carbone.

4. Procédé selon la revendication 1, caractérisé en ce qu'on effectue la réaction en présence d'un solvant inerte.

5. Procédé selon la revendication 1, caractérisé en ce qu'on effectue la réaction avec des aldoximes de formule générale V

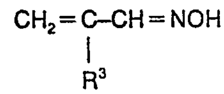

(V),

dans laquelle $R^3$ est un reste alkyle linéaire de 2 à 15 atomes de carbone ou un reste alkyle ramifié ou cyclique ayant jusqu'à 15 atomes de carbone, qui peut éventuellement être en outre substitué.